Europäisches Patentamt

European Patent Office

Office européen des brevets

(19)

(11) Publication number: 0 569 369 B1

# EUROPEAN PATENT SPECIFICATION

(12)

(45) Date of publication of patent specification: 01.03.95    (51) Int. Cl.⁶: C07J 71/00

(21) Application number: 92901023.9

(22) Date of filing: 17.12.91

(86) International application number:
PCT/PL91/00016

(87) International publication number:
WO 92/11280 (09.07.92 92/17)

(54) METHOD OF OBTAINING (22R) DIASTEREOISOMER OF BUDESONIDE.

(30) Priority: 20.12.90 PL 288360

(43) Date of publication of application:
18.11.93 Bulletin 93/46

(45) Publication of the grant of the patent:
01.03.95 Bulletin 95/09

(84) Designated Contracting States:
AT BE CH DE DK ES FR GB IT LI NL SE

(56) References cited:
EP-A- 0 164 636
US-A- 3 996 359

(73) Proprietor: ALCO CHEMICALS LTD.
Swiss Branch
Via San Salvatore 7
CH-6902 Lugano (CH)

(72) Inventor: USZYCKA-HORAWA, Teresa
ul. Etiudy Rewolucyjnej 5/7 m 72
02-643 Warszawa (PL)
Inventor: SMOLINSKA, Jadwiga
pl. Wilsona 4 m 89
01-626 Warszawa (PL)
Inventor: KROSZCZYNSKI, Wojciech
ul. Potocka 6 m 76
01-652 Warszawa (PL)

(74) Representative: Minoja, Fabrizio et al
Studio Consulenza Brevettuale,
Via Rossini, 8
I-20122 Milano (IT)

Rank Xerox (UK) Business Services
(3.10/3.09/3.3.3)

EP 0 569 369 B1

## Description

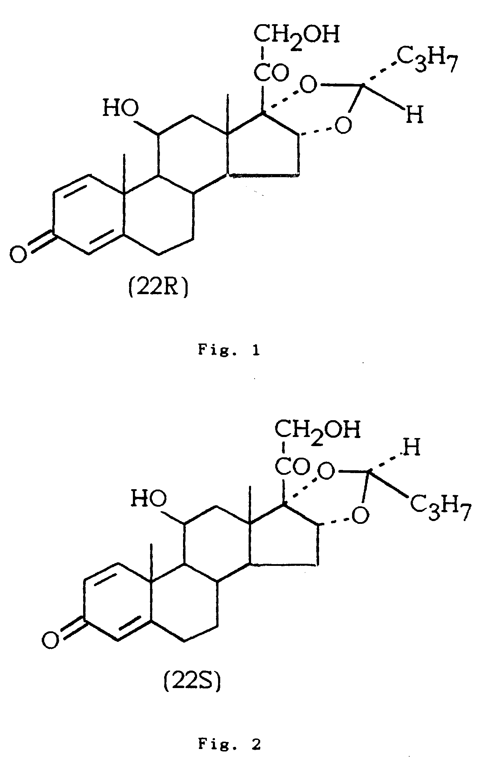

The subject of the invention is a method of obtaining diastereoisomer of (22R) budesonide. Budesonide, i.e. (22R,S)-16α,17α-butylidenedioxy-11β,21-dihydroxy-1,4-pregnadiene-3,20-dione is a mixture of diastereoisomers (22R) and (22S) differing in the position of an acetal chain (fig.1 and 2). Both compounds are active glucocorticoids applied in a mixture (1:1) in pharmaceutical forms: antiasthmatic aerosol or antiallergic ointment. (22R) diastereoisomer is 2-3 times more active pharmacologically than (22S) diastereoisomer [Brattsand R.; Eur.J.Res.Dis.63, suppl. 122,62-73 (1982)].

In pharmacotherapy a tendency is presently observed to apply optically active compounds and not racemic mixtures. In the case of budesonide both isomeric forms are pharmacologically active but their metabolism is different [Andersson P. et al.; Xenobiotica 17,35-44 (1987)]. Testing of action of a drug being a pure chemical individual is considerably easier than that of a mixture.

A known method of obtaining budesonide, that is a mixture of diastereoisomers (22R) and (22S) consists in condensation of 11β,16α,17α,21-tetrahydroxy-1,4-pregnadien-3,20-dione with n-butyric aldehyde in the presence of strong inorganic acids, e.g. perchloric acid, in organic solvents [FRG patent 2323216(1973)].

Those skilled in the art know also a method of obtaining a mixture of diastereoisomers (22R) and (22S), in which isomer (22R) is in majority, even up to 90%. The said method consists in condensation, discussed above, conducted in the presence of hydrofluoric acid of concentration of 48-70% or concentrated hydrochloric acid [Eur.pat.appln.164636 (28.05.85)].

According to the known method separation of a mixture of diastereoisomers (22R) and (22S) is carried out on a column packed with Sephadex LH20 [Thalen A., Nylander B.; 19,247-266(1982)]. The said method requires the use of a very big amount of solvents and long separation time, and for these reasons it is technologically inconvenient, expensive and time-consuming.

Unexpectedly it has appeared out that the product obtained after condensation in the form of 21-acetate containing at least 80% of diastereoisomer (22R), after crystallization from ethanol, and then hydrolyzed and crystallized from ethyl acetate yields pure diastereoisomer (22R) with an admixture of diastereoisomer (22S) of 1% at the very most. The content of diastereoisomers is determined by the HPLC method. From post-crystallization filtrates containing a mixture of diastereoisomers of a composition of 8:2 or 7:3 one may separate budesonide 21-acetate, crystallize it again and after hydrolysis obtain pure diastereoisomer (22R).

By the method according to the invention condensation of 11β,16α,17α,21-tetrahydroxy-1,4-pregnadiene-3,20-dione 21-acetate with n-butyric aldehyde is carried out, in the known way, in the medium of hydrofluoric acid of concentration of 70-80%. The isolated crude condensation product is crystallized from ethanol and obtained 21-acetate of budesonide (22R) of at least 95% content is hydrolyzed, and the product thus obtained is crystallized from ethyl acetate to obtain (22R) diastereoisomer of budesonide of at least 99% content.

An advantageous effect of the invention is that (22R) diastereoisomer of budesonide having the content of at least 99% is obtained at a yield of about 60%. The product obtained by the method according to the invention is an active substance of antiasthmatic aerosol and its content in a dose of 158 μg is equivalent to 200 μg of budesonide of a mixture (1:1) of diastereoisomers (22R and 22S). The method according to the invention is characterized by simplicity of procedure, enables saving organic solvents and is less labour-consuming.

The after-mentioned example illustrates the invention without limiting its scope. Example.

To a mixture of 7 ml of 75% hydrofluoric acid and 0.52 ml of n-butyric aldehyde, cooled to 0°C, 3.5 g of 11β,16α,17α,21-tetrahydroxy-1,4-pregnadiene-3,20-dione 21-acetate is added in and stirred for 3 hours at maintenance of the temperature of 0°C. The obtained dark-red solution is poured into 50 ml of water with ice, neutralized by concentrated ammonia and extracted by chloroform. From the extract chloroform is distilled off under diminished pressure, and the residue is crystallized from ethanol. 2.2 g of 21-acetate of (22R) diastereoisomer of budesonide of $[\alpha]_D^{22}$ +106°- (c=1, $CH_2Cl_2$) is obtained, the content of 21-acetate of (22S) diastereoisomer of budesonide is 5%. The product is suspended in 40 ml of methanol, cooled to 0°C, 2.5 ml of 10% aquas solution of potassium carbonate is added thereto, and is stirred under nitrogen for 1 and 1/2 hours, at maintenance of the temperature of 0°C, after this it is neutralized with acetic acid, methanol is evaporated and (22R) diastereoisomer of budesonide is isolated either by filtering off or by extraction with chloroform. The separated product is crystallized from ethyl acetate and 1.7 g of (22R) diastereoisomer of budesonide is obtained, of melting point of 245-250°C (decomp.) $[a]_D^{22}$ +117.5°C (c=1, $CH_2Cl_2$);

$$a_{1cm}^{1\%}$$

350 at 242 nm. The content of (22S) diastereoisomer of budesonide determined by the the HPLC method is 1%.

## Claims

1. Method of obtaining of (22R) diastereoisomer of budesonide, characterized in that a mixture of budesonide 21-acetate having at least 80% of diastereoisomer 22R is crystallized from ethanol and obtained 21-acetate of budesonide (22R) of at least 95% content is hydrolyzed, and the product thus obtained is crystallized from ethyl acetate.

2. A process for the preparation of (22R)-budesonide acetate characterized in that a mixture of budesonide acetate having at least 80% of diastereoisomer (22R) is crystallized from ethanol to give budesonide acetate having at least 95% of diastereoisomer (22R).

## Patentansprüche

1. Verfahren zum Erhalten des (22R)-Diastereoisomers von Budesonid, dadurch **gekennzeichnet,** daß man eine Mischung aus Budesonid 21-Acetat, das wenigstens 80 % des Diasteroisomer 22R enthält, aus Ethanol kristallisiert, und das erhaltene 21-Acetat von Budesonid (22R) mit einem Gehalt Von wenigstens 95 % hydrolisiert, und das so erhaltene Produkt aus Ethylacetat kristallisiert.

2. Verfahren zur Herstellung des (22R)-Budesonidacetats, dadurch **gekennzeichnet**, daß man eine Mischung des Budesonidacetats, das wenigstens 80 % des Diastereoisomers (22R) enthält, aus Ethanol kristallisiert unter Erhalt von Budesonidacetat mit wenigstens 95 % des Diastereoisomers (22R).

## Revendications

1. Procède d'obtention du diastéréoisomère (22R) de budésonide, caractérisé par le fait qu'un mélange de budésonide 21-acétate comprenant au moins 80% de diastéréoisomère 22R est cristallisé dans l'éthanol et le 21-acétate de budésonide (22R) obtenu avec une teneur d'au moins 95% est hydrolysé, et le produit ainsi obtenu est cristallisé dans de l'acétate d'éthyle.

2. Procédé de préparation de (22R)-budésonide acétate, caractérisé par le fait qu'un mélange de budésonide acétate comprenant au moins 80% de diastéréoisomère (22R) est cristallisé dans de l'éthanol pour donner du budésonide acétate comprenant au moins 95% de diastéréoisomère (22R).

(22R)

Fig. 1

(22S)

Fig. 2

4